# EUROPÄISCHE PATENTSCHRIFT

(11) **EP 1 251 826 B1**
(45) Veröffentlichungstag und Bekanntmachung des Hinweises auf die Patenterteilung: **23.07.2003**
(21) Anmeldenummer: 01903657.3
(22) Anmeldetag: 18.01.2001
(51) Int. Cl.: A61K 9/00, A61K 9/10, A61P 27/06, A61K 47/30

(54) **DICLOFENAMID SUSPENSIONSGEL**
DICLOFENAMIDE SUSPENSION GEL
GEL SUSPENSION DE DICLOFENAMIDE

(30) Priorität: 20.01.2000 DE 10002233
(43) Veröffentlichungstag der Anmeldung: 30.10.2002
(73) Patentinhaber: Dr. GERHARD MANN chem.-pharm. Fabrik GmbH, D-13581 Berlin (DE)
(72) Erfinder: CLAUS-HERZ, Gudrun, 14167 Berlin (DE); BELLMANN, Günther, 13593 Berlin (DE)
(74) Vertreter: Maiwald Patentanwalts GmbH
(86) Internationale Anmeldenummer: EP0100557
(87) Internationale Veröffentlichungsnummer: WO01052812

(56) Entgegenhaltungen:
- US-A- 5 958 443
- FRIDRIKSDOTTIR H ET AL: "Formulation and testing of methazolamide cyclodextrin eye drop solutions" JOURNAL OF CONTROLLED RELEASE,NL,ELSEVIER SCIENCE PUBLISHERS B.V. AMSTERDAM, Bd. 44, Nr. 1, 1. Februar 1997 (1997-02-01), Seiten 95-99, XP004015270 ISSN: 0168-3659
- LOTTI ET AL.: GRAEFE'S ARCHIVE CLIN. EXPERIMENT. OPHTHALMOL., Bd. 222, 1984, Seiten 13-19, XP000995226 in der Anmeldung erwähnt

## Beschreibung

Die vorliegende Erfindung betrifft eine neue ophthalmische Zusammensetzung in Form eines Suspensionsgels, die Diclofenamid in Form der freien Säure enthalten. Ferner betrifft die vorliegende Erfindung die Verwendung von Diclofenamid Suspensionsgel zur Herstellung eines Arzneimittels zur Behandlung von okulärer Hypertension und zur Therapie von primären und sekundären Offenwinkel-Glaukomen.

Die Verwendung von Diclofenamid (INN: Dichlorphenamid, 4,5-Dichlorbenzol-1,3-Disulfonamid) zur Senkung des intraokularen Drucks ist seit langem bekannt. Die freie Säure des Diclofenamids wird im Stand der Technik nahezu ausschließlich systemisch verabreicht. Diclofenamid wirkt als Carboanhydrasehemmer, welches das multifunktionelle Enzym Carboanhydrase blockiert. Im Auge wird Carboanhydrase (II) reversibel inhibiert, was letztlich zu einer Verringerung der wäßrigen Sekretion und folglich zur Absenkung des Augeninnendrucks führt.

Bei der Behandlung von Glaukomen, einer degenerativen Erkrankung des Auges, die mit einem Anstieg des Augeninnendrucks einhergeht, werden Carboanhydrasehemmer wie Diclofenamid eingesetzt. Neben der systemischen Applikation von Diclofenamid sind seit den 80-iger Jahren auch Präparate zur topischen Anwendung bekannt geworden und finden zunehmend Verbreitung. Während zur systemischen Verabreichung meist die freie Säure des Didofenamids eingesetzt wird, sind zur topischen Anwendung bisher lediglich die Alkalimetallsalze des Diclofenamids eingesetzt worden.

Insbesondere die salzbildenden Carboanhydrasehemmer haben in ophthalmischen Zusammensetzungen zur topischen Anwendung bei der Behandlung okulärer Hypertension Anwendung gefunden.

So beschreibt beispielsweise die europäische Patentanmeldung EP-A-033 042 die Verwendung diverser Carboanhydrasehemmer in Form ihrer Alkalimetallsalze zur Herstellung von ophthalmischen Präparaten. Neben Beispielen für ophthalmische Lösungen von Methazolamid-Natriumsalz, teilweise gelartig verdickt mit Hydroxypropylmethylcellulose, wird in der EP-A-033 042 u.a. auch die Verwendung von Diclofenamid-Dikaliumsalz beschrieben.

Die Verwendung von Monoalkalimetallsalzen dibasischer Carboanhydrasehemmer in ophthalmischen Zusammensetzungen zur topischen Anwendung wird in der europäischen Patentanmeldung EP-A-036 351 beschrieben. Die entsprechenden ophthalmischen Zusammensetzungen sind Lösungen des Wirkstoffs bzw. Lösungsgele.

US 5,958,443 beschreibt ophthalmische Wirkstoff-Gele, die DicloFenamid enthalten können.

Lotti et al. (Graefe's Archive for Clinical and Experimental Ophthalmology 1984, 222:13-19) haben eine Studie zur topischen okulären hypotensiven Aktivität und okulären Penetration von Diclofenamid-Natrium bei Kaninchen durchgeführt. Dabei wurde festgestellt, daß eine wäßrige Lösung des Natriumsalzes von Diclofenamid in der Lage ist, eine deutliche und langanhaltende Absenkung des Augeninnendrucks herbeizuführen. Lotti et al. berichten, daß die Verwendung von Lösungen von Diclofenamid-Natriumsalz gegenüber Suspensionen der freien Säure des Diclofenamids zu einem deutlich stärkeren und längeren Absenkung des Augeninnendrucks führt.

Auch die Kammerwasserwerte waren in der Studie von Lotti et al. bei der Anwendung Suspensionen des Diclofenamids gegenüber Lösungen des Natriumdiclofenamids auf deutlich niedrigerem Niveau. Allerdings wurde von Lotti et al. auch festgestellt, daß obwohl einzelne Anwendungen von Natriumdiclofenamidlösungen gut toleriert werden, die wiederholte Anwendung von Diclofenamid-Natriumlösungen schwere unerwünschte Nebenwirkungen bei Kaninchen auslöst.

Zur Überwindung dieser bekannten Nachteile der ophthalmischen Diclofenamid enthaltenden Zusammensetzungen des Standes der Technik liegt der vorliegenden Erfindung die Aufgabe zugrunde, eine Diclofenamid enthaltende ophthalmische Zusammensetzung zur Verfügung zu stellen, welche in effektiver Weise den Augeninnendruck abzusenken in der Lage ist, und dabei gute Verträglichkeit aufweist.

Eine weitere Aufgabe der Erfindung liegt darin, ein Suspensionsgel der freien Säure des Diclofenamids zur Verfügung zu stellen, das anstelle von Diclofenamid-Zubereitungen zur systemischen Verabreichung verwendet werden kann.

Die vorliegende Erfindung löst die oben genannten Aufgaben durch Bereitstellung einer ophthalmischen Zusammensetzung zur Glaukombehandlung in Form eines Suspensionsgels, umfassend die freie Säure des Diclofenamids, Konservierungsmittel, Gelbildner, Mittel zur Einstellung des pH-Wertes, Wasser sowie gegebenenfalls weitere übliche Zusatzstoffe.

Überraschenderweise konnte im Gegensatz zu der Studie von Lotti et al. gefunden werden, daß die Formulierung von Diclofenamid als freier Säure in Suspensionsgelen zu ophthalmischen Präparaten führt, welche vergleichbare Effekte zu denen erzielen können, die durch die systemische Verabreichung von Diclofenamid erreicht werden können.

Die Formulierung von enthaltenden ophthalmischen Zusammensetzungen mit Diclofenamid in Form der freien Säure als Suspensionsgele gemäß der vorliegenden Erfindung führt zu hochwirksamen und gut verträglichen Therapeutika zur Behandlung von primären und sekundären Offenwinkelglaukomen sowie okulärer Hypertension.

Zur Herstellung der erfindungsgemäßen Suspensionsgele muß die freie Säure des Diclofenamids mikronisiert werden. Diese Mikronisierung erfolgt nach den im Stand der Technik üblichen Verfahren, zum Beispiel durch Malen in verschiedenen Arten von Mühlen, Verreiben und Sieben.

Die mikronisierten Partikel zur Verwendung in erfindungsgemäßen ophthalmischen Zusammensetzungen weisen vorzugsweise Partikelgrößen von weniger als 90 µm, vorzugsweise weniger als 50 µm, besonders bevorzugt kleiner 25 µm, auf.

Vorzugsweise weisen mehr als 50 %, bevorzugt mehr als 60 %, bevorzugt mehr als 80 % und am meisten bevorzugt mehr als 99% der Diclofenamidpartikel eine Partikelgröße von weniger als 90 µm auf.

Erfindungsgemäß geeignet weisen mehr als 50 %, vorzugsweise mehr als 60 %, bevorzugt mehr als 80 % und noch bevorzugter mehr als 95% der Diclofenamidpartikel eine Partikelgröße von weniger als 50 µm auf.

Vorteilhaft ist, wenn mehr als 10 %, vorzugsweise mehr als 20 %, noch bevorzugter mehr als 30 % und noch bevorzugter mehr als 50 %, weiter bevorzugt mehr als 80% und am meisten bevorzugt mehr als 95% der Diclofenamidpartikel eine Partikelgröße von kleiner als 25 µm aufweisen.

Insbesondere bevorzugt ist wenn 100% der Diclofenamidpartikel kleiner als 90 µm, noch bevorzugter kleiner 50 µm und am meisten bevorzugt kleiner 25 µm ist.

Vor der Weiterverarbeitung zum Suspensionsgel wird das mikronisierte Diclofenamid vorzugsweise sterilisiert.

Als Gelbildner können erfindungsgemäß alle für die Herstellung stabiler Suspensionsgele geeigneten Polymerverbindungen verwendet werden. Insbesondere wird der Gelbildner ausgewählt aus der Gruppe bestehend aus Polyacrylsäure, Celluloseether, Polyvinylalkohol und Polysacchariden. Diese können wahlweise synthetischen oder natürlichen Ursprungs sein.

Besonders bevorzugt ist die Verwendung von Hydrogelen auf der Basis von Polyacrylsäure und/oder Celluloseethern. Insbesondere bevorzugt sind Polyacrylsäuregele, insbesondere auf der Basis von Carbomeren des Carbopol®typs, kommerziell erhältlich von B.F.Goodrich. Besonders bevorzugt ist die Verwendung von Carbopol 980 als Gelbildner in erfindungsgemäßen Suspensionsgelen.

In bevorzugten Ausführungsformen der erfindungsgemäßen Diclofenamid-Suspensionsgele sind Polyacrylsäuregele, wie Carbopol, insbesondere Carbopol 980, mit einem Gehalt von >0 bis 10 Gew.-%, bevorzugt 0,01 Gew.-% - 5 Gew.-%, noch bevorzugter 0,05 Gew.-% - 1 Gew.-%, weiter bevorzugt 0,1 Gew.-% - 0,5 Gew.-% und am meisten bevorzugt 0,2 Gew.-% - 0,4 Gew.-% enthalten.

Um eine eventuelle Kontamination des Gels während der Applikation zu verhindern, werden die erfindungsgemäßen ophthalmischen Zusammensetzungen durch Zusatz eines Konservierungsmittels konserviert. Als Konservierungsmittel können alle zur Anwendung am Auge geeigneten, dem Fachmann wohlbekannten Stoffe verwendet werden, beispielsweise Benzylalkohol, Benzalkoniumchlorid oder andere quarternäre Ammoniumverbindungen, Chlorhexidindiacetat oder -dicluconat, Thiomersal etc. Besonders bevorzugt ist die Verwendung von Benzododeciniumsalzen, insbesondere Benzododeciniumchlorid.

Als weiterer Zusatzstoff wird in den erfindungsgemäßen Zusammensetzungen vorzugsweise Sorbitol verwendet. In bevorzugten Ausführungsformen der erfindungsgemäßen Diclofenamid-Suspensionsgele ist Sorbitol und/oder ein Polyacrylsäuregel mit einem Gehalt von 0 bis 10 Gew.-%, bevorzugt 2 bis 8 Gew.-%, besonders bevorzugt 3 bis 6 Gew.-% und am meisten bevorzugt zwischen von 4 Gew.-% und 5 Gew.-% enthalten.

Anstelle von Sorbitol kann auch Glycerol, Mannitol und/oder Dextrose verwendet werden.

Zur Einstellung des pH-Wertes der erfindungsgemäßen Suspensionsgele werden mineralische Säuren und Basen verwendet. Besonders bevorzugt ist die Verwendung von Natriumhydroxid und/oder Salzsäure.

Der pH-Wert der erfindungsgemäßen ophthalmologischen Zusammensetzung liegt zwischen von pH 6,8 - pH 7,6, vorzugsweise von zwischen pH 7 - pH 7,5, bevorzugt von zwischen pH 7,1-pH 7,5 und am meisten bevorzugt von zwischen pH 7,25-pH 7,35.

Bevorzugte Ausführungsformen der erfindungsgemäßen Suspensionsgele weisen eine Viskosität zwischen 1.000 bis 8.000 mPa·s, bevorzugt zwischen 2.000 und 6.000 mPa·s und besonders bevorzugt zwischen 2.500 und 5.500 mPa·s und noch bevorzugter zwischen 2.800 und 4.500 mPa·s auf.

Vorzugsweise wird die Viskosität der erfindungsgemäßen Gelzusammensetzung so eingestellt, daß die Kontaktzeit am Auge ≥ 1 min., insbesondere wenigstens ≥ 5 min., vorzugsweise ≥ 10 min., weiter bevorzugt ≥ 15 min., noch bevorzugter ≥ 20 min. und am meisten bevorzugt ≥ 30 min. beträgt. Die Kontaktzeit ist der Zeitraum, gemessen ab Auftragung des erfindungsgemäßen Gels bis zu dem Zeitpunkt, ab dem das Gel vollständig zusammen mit Tränenflüssigkeit vom Auge weggespült worden ist.

Der Wirkstoffgehalt der erfindungsgemäßen Suspensionsgele liegt bei 0,1 bis 10 Gew.-%, bevorzugt 0,1 bis 5 Gew.-%, weiter bevorzugt 0,5 Gew.-%- 4 Gew.-%, noch bevorzugter 1 Gew.-% - 3 Gew.-% und am meisten bevorzugt 1,5 Gew.-%-2 Gew.-% der freien Säure des Diclofenamids.

Noch bevorzugter sind Suspensionsgele, die einen Wirkstoffgehalt von zwischen 2 Gew.-% - 7 Gew.-% der freien Säure des Diclofenamids aufweisen.

Ein Vorteil der erfindungsgemäßen Suspensionsgele der freien Säure des Diclofenamids gegenüber den im Stand der Technik bekannten Lösungen und Lösungsgelen der Diclofenamid-Kalium- bzw. Natriumsalze ist insbesondere dessen deutlich bessere Verträglichkeit. Es treten beispielsweise keine unerwünschten Augenreizungen wie Brennen und dergleichen auf.

Die Herstellung der erfindungsgemäßen Suspensionsgele erfolgt nach den üblichen Verfahren des Standes der Technik. Der mikronisierte Wirkstoff wird sterilisiert und einer sterilen Suspensionsgel-Matrix zugesetzt. Anschließend wird so homogenisiert, daß eine völlig gleichmäßige Verteilung der suspendierten Partikel erreicht wird.

Die erfindungsgemäßen ophthalmischen Diclofenamid-Zusammensetzungen eignen sich zur drucksenkenden Therapie von primären und sekundären Offenwinkelglaukomen sowie zur Behandlung okulärer Hypertension.

Die Verträglichkeit der erfindungsgemäßen Gelformulierungen, umfassend die freie Säure des Diclofenamids, über einen Verabreichungszeitraum von 6 Wochen ist sehr gut, d.h., es treten keine unerwünschten Nebenwirkungen am Auge auf.

Gewichtsangaben, wenn nicht anders angegeben, beziehen sich auf die Gesamtzusammensetzung der erfindungsgemäßen Gelzusammensetzung.

Suspensionsgele im Sinne dieser Erfindung sind Gele, bei denen der Wirkstoff in ungelöster Form als Feststoffpartikel fein suspendiert in der Gelgrundlage bzw. Trägermatrix enthalten ist.

Im folgenden wird die Erfindung anhand einiger Ausführungsbeispiele näher erläutert.

### Beispiele 1 bis 6

Es wurden 6 Diclofenamid Suspensionsgele mit Konzentrationen von 0,1 bis 5 Gew.-% der freien Säure des Diclofenamids hergestellt. Die Zusammensetzung der einzelnen Beispiele sind in der nachfolgenden Tabelle 1 wiedergegeben. Alle Prozentangaben verstehen sich als Gewichtsprozent bezogen auf die Gesamtzusammensetzung.

Die hergestellten Suspensionsgele weisen die in Tabelle 2 dargestellten physikalischen Eigenschaften auf:

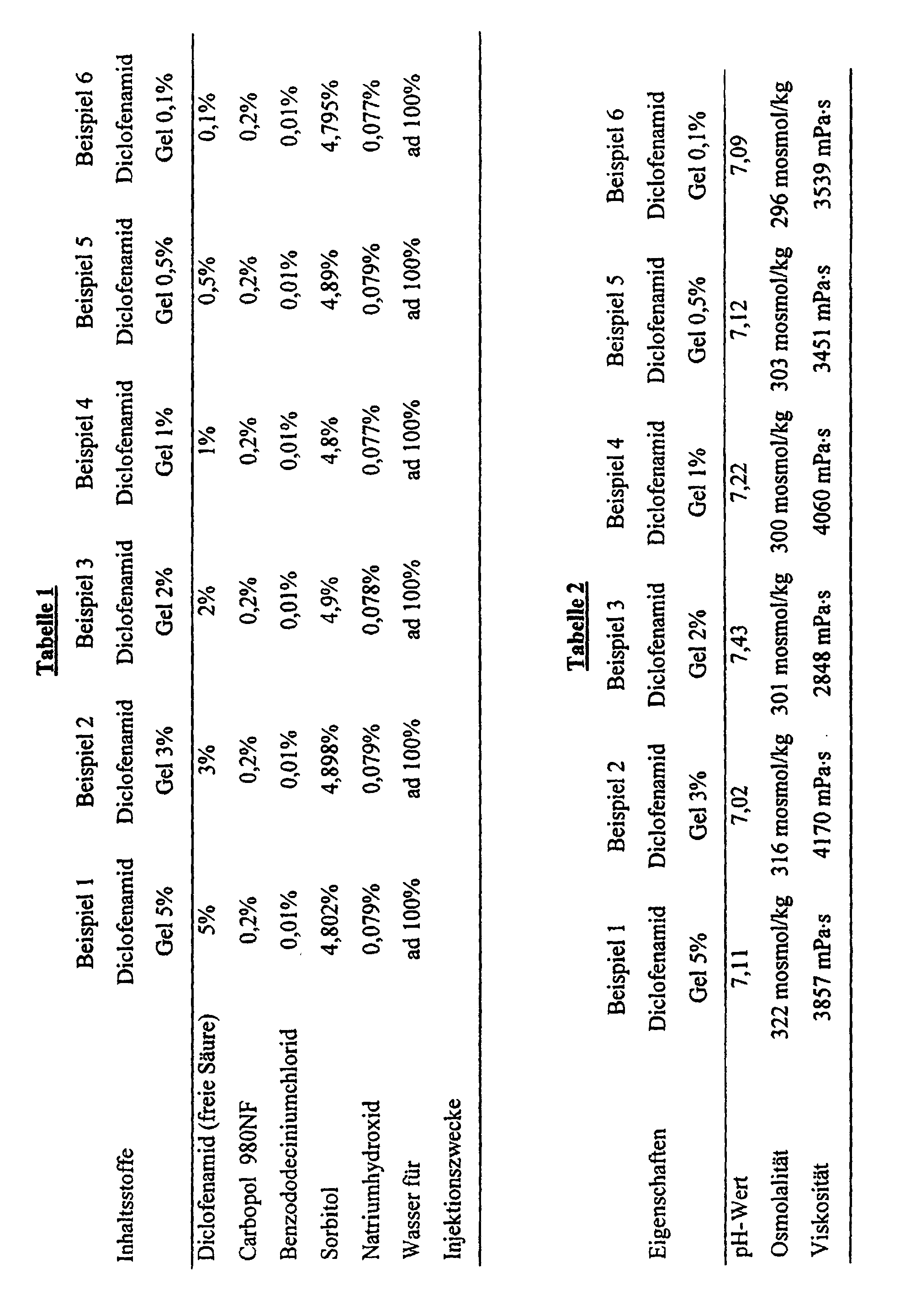

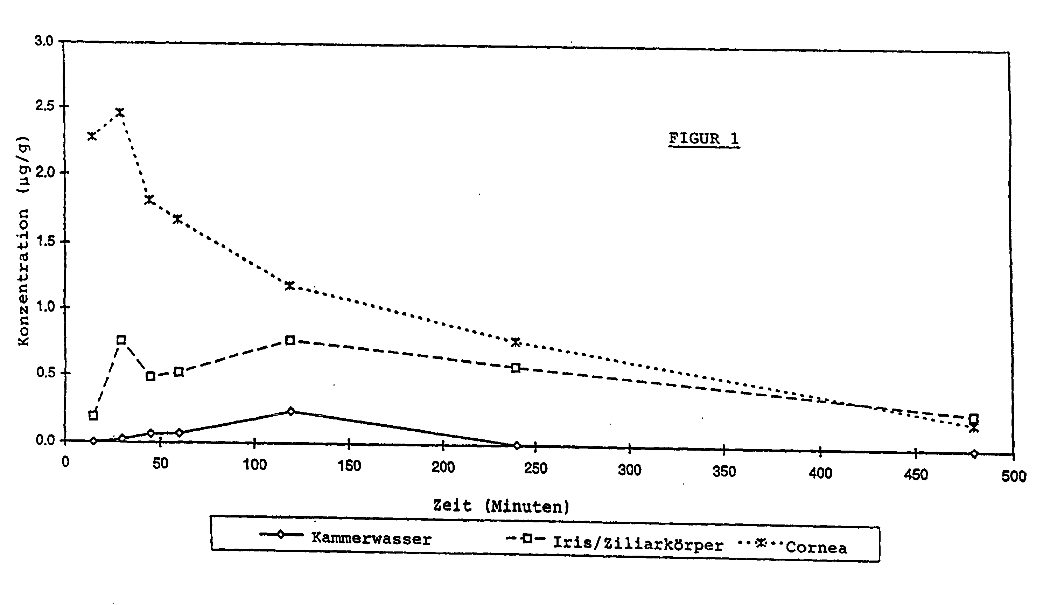

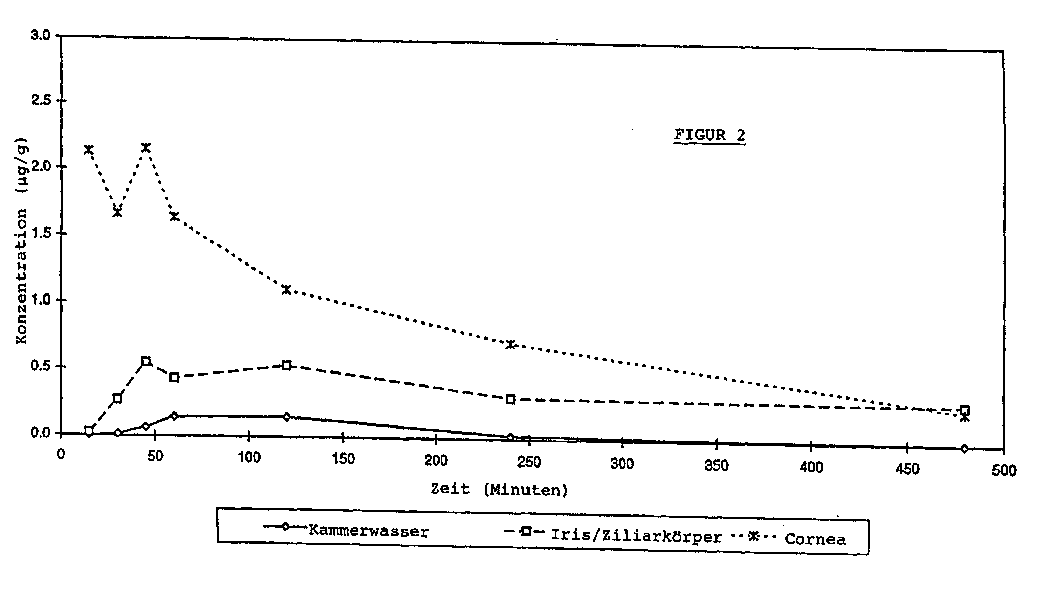

Mit den 3 und 5-%igen Gelen aus Beispiel 1 und 2 wurde eine intraokulare Bioverfügbarkeitsstudie der Einzeldosis bei Kaninchen durchgeführt. Hierzu wurden nach verschiedenen Zeitabständen die Konzentrationen von Diclofenamid im Kammerwasser im Iris-Ziliarkörper an der Cornea gemessen. Die gemessenen Konzentrationen sind in den Figuren 1 und 2 gegen die Zeit aufgetragen.

Hierbei zeigt
- Figur 1: die Konzentrationszeitkurve von Diclofenamid bei Anwendung des Suspensionsgels aus Beispiel 1, und
- Figur 2: die Konzentrationszeitkurve von Diclofenamid bei Anwendung des Suspensionsgels aus Beispiel 2.

Den Figuren 1 und 2 ist zu entnehmen, daß die Durchdringungsfähigkeit der freien Säure des Diclofenamids aus dem Suspensionsgel durch die Cornea relativ groß ist. Im Vergleich zur systemischen Gabe liegen die Iris-Ziliarkörperspiegel des aus dem erfindungsgemäßen Suspensionsgel topisch angewendeten Diclofenamids über 8 Stunden auch auf vergleichbarem Niveau.

Die Verträglichkeit der getesteten Gelformulierungen ist über einen Versuchszeitraum von 6 Wochen sehr gut bei der 3 x täglichen Anwendung der getesteten Suspensionsgele über einen Zeitraum von 6 Wochen konnten keinerlei Augenreizungen oder Gewebeschädigungen des Auges festgestellt werden. Ebenso wurden keine Anzeichen für systemische Nebenwirkungen gefunden.

## Patentansprüche

1. Ophthalmische Zusammensetzung zur Glaukombehandlung in Form eines Suspensionsgels, umfassend die freie Säure des Diclofenamids, Konservierungsmittel, Gelbildner, Mittel zur Einstellung des pH-Wertes, Wasser sowie gegebenenfalls weitere übliche Zusatzstoffe.

2. Ophthalmische Zusammensetzung nach Anspruch 1,
**dadurch gekennzeichnet, daß** der Gelbildner aus der Gruppe bestehend aus Polyacrylsäure, Celluloseether, Polyvinylalkohol und Polysacchariden ausgewählt ist.

3. Opthalmische Zusammensetzung nach einem der Ansprüche 1 oder 2,
**dadurch gekennzeichnet, daß** als Konservierungsmittel Benzododeciniumchlorid verwendet wird.

4. Opthalmische Zusammensetzung nach einem der Ansprüche 1 bis 3,
**dadurch gekennzeichnet, daß** als Gelbildner Carbopol, vorzugsweise Carbopol 980 verwendet wird.

5. Ophthalmische Zusammensetzung nach einem der Ansprüche 1 bis 4,
**dadurch gekennzeichnet, daß** das Suspensionsgel zwischen 0,1 bis 10 Gew.-% und bevorzugt 0,1 bis 5 Gew.-% der freien Säure des Diclofenamids enthält.

6. Ophthalmische Zusammensetzung nach einem der Ansprüche 1 bis 5,
**dadurch gekennzeichnet, daß** das Suspensionsget 0 bis 10 Gew.-%, bevorzugt 2 bis 8 Gew.-% und besonders bevorzugt 3 bis 6 Gew.-% Sorbitol enthält.

7. Ophthalmische Zusammensetzung nach einem der Ansprüche 1 bis 6,
**dadurch gekennzeichnet, daß** die Viskosität des Suspensionsgels zwischen 1.000 bis 8.000 mPa·s, bevorzugt zwischen 2.000 und 6.000 mPa·s und besonders bevorzugt zwischen 2.500 und 5.500 mPa·s liegt.

8. Ophthalmische Zusammensetzung nach einem der Ansprüche 1 bis 7,
**dadurch gekennzeichnet, daß** das Diclofenamid bis auf Partikelgrößen von kleiner als 90 um, vorzugsweise kleiner als 50 µm, besonders bevorzugt kleiner 25 µm, mikronisiert ist.

9. Verwendung einer ophthalmischen Zusammensetzung nach einem der Ansprüche 1 bis 8 zur Herstellung eines Arzneimittels zur Senkung des Augeninnendrucks.

10. Verwendung einer ophthalmischen Zusammensetzung nach einem der Ansprüche I bis 8 zur Herstellung eines Arzneimittels zur Behandlung von primären und/oder sekundären Offenwinkelglaukomen.

11. Verwendung einer ophthalmischen Zusammensetzung nach einem der Ansprüche 1 bis 8 zur Herstellung eines Arzneimittels zur Behandlung okulärer Hypertension.

## Claims

1. Ophthalmic composition in the form of a suspension gel for treating glaucoma, comprising the free acid of diclofenamide, preservative, gel former, means for adjusting the pH, water and optionally further conventional additives.

2. Ophthalmic composition according to claim 1, **characterised in that** the gel former is selected from the group consisting of polyacrylic acid, cellulose ether, polyvinyl alcohol and polysaccharides.

3. Ophthalmic composition according to either of claims 1 or 2, **characterised in that** benzododecinium chloride is used as the preservative.

4. Ophthalmic composition according to any one of claims 1 to 3, **characterised in that** Carbopol, preferably Carbopol 980 is used as the gel former.

5. Ophthalmic composition according to any one of claims 1 to 4, **characterised in that** the suspension gel contains between 0.1 to 10 % by weight and preferably 0.1 to 5 % by weight of the free acid of diclofenamide.

6. Ophthalmic composition according to any one of claims 1 to 5, **characterised in that** the suspension gel contains 0 to 10 % by weight, preferably 2 to 8 % by weight and particularly preferably 3 to 6 % by weight sorbitol.

7. Ophthalmic composition according to any one of claims 1 to 6, **characterised in that** the viscosity of the suspension gel is between 1,000 to 8,000 mPa·s, preferably between 2,000 and 6,000 mPa·s and particularly preferably between 2,500 and 5,500 mPa·s.

8. Ophthalmic composition according to any one of claims 1 to 7, **characterised in that** the diclofenamide is micronised to particle sizes of smaller than 90 µm, preferably smaller than 50 µm, particularly preferably smaller than 25 µm.

9. Use of an ophthalmic composition according to any one of claims 1 to 8, for producing a medicinal drug for reducing intra-ocular pressure.

10. Use of an ophthalmic composition according to any one of claims 1 to 8, for producing a medicinal drug for treating primary and/or secondary open-angle glaucomas.

11. Use of an ophthalmic composition according to any one of claims 1 to 8, for producing a medicinal drug for treating ocular hypertension.

## Revendications

1. Composition ophtalmique pour le traitement du glaucome sous la forme d'un gel en suspension, comprenant l'acide libre du diclofénamide, des agents de conservation, des agents de gélification, des agents pour régler la valeur de pH, de l'eau, ainsi que le cas échéant d'autres additifs habituels.

2. Composition ophtalmique selon la revendication 1, **caractérisée en ce que** l'agent de gélification est choisi parmi le groupe constitué par de l'acide polyacrylique, de l'éther cellulosique, de l'alcool polyvinylique et des polysaccharides.

3. Composition ophtalmique selon l'une quelconque des revendications 1 et 2, **caractérisée en ce qu'**on utilise, à titre d'agent de conservation, du chlorure de benzododécinium.

4. Composition ophtalmique selon l'une quelconque des revendications 1 à 3, **caractérisée en ce qu'**on utilise, à titre d'agent de gélification, du Carbopol, de préférence du Carbopol 980.

5. Composition ophtalmique selon l'une quelconque des revendications 1 à 4, **caractérisée en ce que** le gel en suspension contient l'acide libre du diclofénamide à concurrence d'une valeur entre 0,1 à 10 % en poids, et de préférence de 0,1 à 5 % en poids.

6. Composition ophtalmique selon l'une quelconque des revendications 1 à 5, **caractérisée en ce que** le gel en suspension contient du sorbitol à concurrence d'une valeur entre 0 à 10 % en poids, de préférence de 2 à 8 % en poids, et de manière particulièrement préférée de 3 à 6 % en poids.

7. Composition ophtalmique selon l'une quelconque des revendications 1 à 6, **caractérisée en ce que** la viscosité du gel en suspension se situe entre 1000 et 8000 mPa.s, de préférence entre 2000 et 6000 mPa.s, et de manière particulièrement préférée entre 2500 et 5500 mPa.s.

8. Composition ophtalmique selon l'une quelconque des revendications 1 à 7, **caractérisée en ce que** le diclofénamide est soumis à une micronisation pour obtenir des granulométries inférieures à 90 µm, de préférence inférieures à 50 µm, de manière particulièrement préférée inférieures à 25 µm.

9. Utilisation d'une composition ophtalmique selon l'une quelconque des revendications 1 à 8, pour la préparation d'un médicament destiné à abaisser la pression intraoculaire.

10. Utilisation d'une composition ophtalmique selon l'une quelconque des revendications 1 à 8, pour la préparation d'un médicament destiné à traiter des glaucomes primaires et/ou secondaires à angle ouvert.

11. Utilisation d'une composition ophtalmique selon l'une quelconque des revendications 1 à 8, pour la préparation d'un médicament destiné à traiter l'hypertension oculaire.
